# EUROPEAN PATENT SPECIFICATION

(11) **EP 1 627 725 B1**
(45) Date of publication and mention of the grant of the patent: **17.12.2008**
(21) Application number: 05017564.5
(22) Date of filing: 12.08.2005
(51) Int. Cl.: B29C 59/14, B29C 59/16, B29C 71/04, C08J 7/14, G01N 33/49, G01N 11/16

(54) **Method of treating the surface of a medical device for blood coagulation diagnosis, and such a device**
Verfahren zur Oberflächenbehandlung einer medizinischen Vorrichtung für die Untersuchung der Blutgerinnungsfunktion, sowie eine dergestalte Vorrichtung
Procédé de traitement de surface d'un dispositif médical pour le diagnostic de la coagulation sanguine, et un tel dispositif

(30) Priority: 12.08.2004 GB 0418474
(43) Date of publication of application: 22.02.2006
(73) Proprietor: C A Casyso AG, 4132 Muttenz (CH)
(72) Inventor: Schubert, Axel, 81377 München (DE); Wurlitzer, Stefan, 85630 Grasbrunn (DE); Glauner, Martin, 79639 Grenzach-Wyhlen (DE); Calatzis, Andreas, 81673 München (DE)
(74) Representative: Rentsch & Partner

(56) References cited:
- EP-A- 0 404 456
- EP-A- 0 629 445
- WO-A-02/066958
- DE-A1- 10 003 093
- IE-B- 70 604
- US-A- 4 341 111
- US-A- 5 627 079
- US-A- 5 723 219
- US-B1- 6 417 004

## Description

### Technical field

The invention relates to the field of medical applications requiring increased adhesion strengths of clotting or clotted blood or a clot of blood components on artificial surfaces, e.g. for blood coagulation diagnosis (hemostasis analysis) or vascular treatment.

### Background of the invention

It is essential for survival that a wound stops bleeding, i.e. that the body possesses an adequate mechanism for hemostasis. The process of blood clotting can be activated in the case of injuries or inflammations by either extrinsic or intrinsic factors, e.g. tissue factor (TF) or Hagemann factor (F XII), respectively. Both activation channels are continued in a common branch of the cascade resulting in thrombin formation. The thrombin itself finally initiates the formation of fibrin fibers, which represent the protein backbone of blood clots.

Various methods have been introduced to assess the potential of blood to form an adequate clot and to determine the blood clots stability. One group of tests is summarized by the term "viscoelastic methods". The common feature of these methods is that the blood clot firmness (or other parameters dependent on it) is continuously determined, from the formation of the first fibrin fibers, till the dissolution of the blood clot by fibrinolysis. Blood clot firmness is a functional parameter, which is important for haemostasis in vivo, as a clot must resist blood pressure and shear stress at the site of vascular injury. Clot firmness results from multiple interlinked processes: coagulation activation, thrombin formation, fibrin formation and polymerisation, platelet activation and fibrin-platelet interaction and can be compromised by fibrinolysis. Thus, by the use of the viscoelastic monitoring all these mechanisms of the coagulation system can be assessed.

The first viscoelastic method was called "thrombelastography" (Hartert H: Blutgerinnungsstudien mit der Thrombelastographie, einem neuen Untersuchungsverfahren. Klin Wochenschrift 26:577-583, 1948). In the thromboelastography, the sample is placed in a cup that is periodically rotated to the left and right by about 5°. A pin is freely suspended by a torsion wire. When a clot is formed it starts to transfer the movement of the cup to the pin against the reverse momentum of the torsion wire. The movement of the pin is continuously recorded and plotted against time.

The fibrin backbone creates a mechanical, elastic linkage between the two surfaces of the blood-containing cup and a pin plunged therein. A proceeding coagulation process induced by adding one or more activating factor(s) can thus be observed. In this way, various deficiencies of a patient's haemostatic status can be revealed and used for proper medical intervention.

Modifications of the original thromboelastography technique (also called thromboelastometry) have been described by Cavallari et al. (US 4,193,293), by Do et al. (US 4,148,216), by Cohen (US 6,537,819), by Hartert et al. (US 3,714,815) and by Calatzis et al. (US 5,777,215).

A common feature of all the methods used for coagulation diagnosis is that the blood clot is placed between two cylindrical bodies and the ability of the blood clot to couple those two bodies is determined. However, the measurement can only be evaluated as long as the fibrin network is sufficiently bound to the surfaces of these two bodies, i.e. of cup and pin. If the fibers tear off even partly, the disturbed measurement becomes hard to interpret because of interference between this effect and the pathologic pattern of hyperfibrinolysis. Unfortunately, such tear-offs of the fibrin network can occur in the case of increased thrombocyte concentrations as observable for example in the blood of haematologic patients (thrombocytosis). In these patients the strength of the blood clot is enhanced and this can lead to too strong forces on the plastic surface, which can tear the clot off the material. For that reason, an enhancement of the blood-clot adhesion strength would improve the therapeutic security considerably.

The original cup and pin material used for thromboelastometry during the fourties until the seventies was stainless steel. These cups and pins were cleaned between measurements and reused.

When thromboelastometry was developed most devices used in the medical laboratory were reused and were usually made of steel, other metals, glass or other durable materials. During the sixties and seventies most of these devices have been exchanged by disposable items made of plastic. These plastic parts are usually economically produced by injection moulding or equivalent techniques.

Disposable cups and pins for the use in thromboelastometry have been described in US 4,148,216 by Do et al and in US 5,223,227 by Zuckerman. In the US 5,223,227 a production process for the cup and pin material is disclosed, which involves a roughening process of the moulds used for the injection moulding of the cups and pins. Roughening of the mould is the common strategy for enhancing the surface roughness of plastic parts produced by injection moulding. In the US 5,223,227 mechanically roughening of the mould by sandblasting is described.

The approach of roughening the mould to roughen the surface of the cup and pin to thereby enhance the adhesion of blood on the surface of the cup and the pin has several disadvantages:

The blood-plastic interaction takes place especially in a microscopic range of the single plastic and of the fibrin molecules. In contrast the roughness produced by the injection moulding process is in a much larger range. Because of this the plastic surfaces produced by injection moulding provide only a limited adhesion of the blood clot. This may be sufficient for the analysis of normal blood, but may be inadequate when factors are present in the blood which compete with the adhesion of fibrin onto the plastic surfaces.

To obtain a reproducible adhesion of blood on a surface it is crucial to have a reproducible surface roughness.

However, the surface roughness of the injection mould changes during long-term use of the mould and also varies when a new mould has to be produced, or when several cavities are used for the production of the same part (in multi-cavity injection moulding tools).

The process for reaching an identical surface roughness on a injection moulding tool compared to previously applied tools can be very expensive and time-consuming. Several surface modifications may need to be performed, sample parts have to be produced and evaluated.

In addition the surface properties produced by injection moulding vary depending on several injection moulding parameters (pressure, temperature) as well as by the batch of the plastic material applied.

The need for improved blood-clot anchoring at artificial surfaces also exists for implants or special artery sealings. In both cases the surface properties must allow a sufficient adhesion of coagulated blood. In the case of implants the blood clot serves as a scaffold for tissue regeneration. Although most polymeric surfaces show in general a good ability to bind blood-inherent coagulation components like thrombocytes and fibrin, the maximum tearing force is not adequate in the case of higher blood pressures or vascular motions. In view of this, improvements of the blood anchoring ability at artificial surfaces enable the use of highly sufficient vascular sealings.

In conclusion a method to improve the adhesion strength of blood clots onto plastic surfaces for diagnostic as well as for biomedical uses is highly desirable.

In the last decade, a rather overwhelming number of plasma applications to enhance the biocompatibility of medical devices in blood contact was disclosed (e.g. US 6,159,531; US 5,591,140; US 5,262,451). These attempts were made to reduce the common blood affinity of artificial surfaces, thus preventing disadvantageous blood clotting and/or tissue deposition. The reported applications contain surgical equipments and in-vivo implants as well as disposables for blood storage or diagnostic purposes.

From US 5,334,611 it is known to treat the surface of e.g. polystyrene parts with plasma to further accelerate the clotting activation of blood in contact to surfaces made from such polymers.

The US 6417004 describes a method and apparatus for detection of a diagnostically significant amount of clot in an aliquot of blood. In a preferred embodiment, said method uses a cocktail of activators to facilitate and proctor absolute activation of Factor XII (the contact factor for the intrinsic pathway of coagulation). Possible activators include Celite(R), kaolin, glass beads, colloidal silica, and diatomaceous material. It is described that this cocktail formulation is important because some individuals respond differently to different activators and hence, the cocktail formulation facilitates standardization of the patient population by reducing the patient-activator sensitivity artifacts.

EP-A-0629445 discloses blood collection tubes made of clot accelerating plastic. An evacuated blood collection assembly includes a plastic container having a plasma-treated inside wall surface which faciliates acceleration of clotting activation. An open end of the container is covered by a puncturable septum. It is describes that the acceleration of clotting activation by the inside wall surface may also be caused by a surface area increased by abraison. It is an crucial feature of the invention according to EP-A-0629445, that the clotting dynamics of the blood samples are affected by the surface treatment.

The object of the present invention is to provide a device, to the surface of which a blood clot adheres with high adhesion strength. It is also an object of the invention to provide a method for preparing a surface such that the adhesion of a blood clot or to a clot of blood components to the surface is enhanced. A further object of the invention is to provide a device for more reliable coagulation analysis. The object is attained by a method of treating

the surface of a device which is to be coupled to a clot of blood or to a clot of blood components according to claim 1, and a device which is to be coupled to a clot of blood or to a clot of blood components according to claim 16.

The method of treating the surface is especially advantageous in that the blood adhesion strength of a clot of blood or blood components on a surface is increased compared with an untreated surface.

The device for coagulation diagnosis has the advantage over the prior art that coagulation properties of blood or blood components can be measured reliably also in case of abnormal blood properties which is the crucial purpose of such a device.

### Brief description of the drawings

In the Figures:

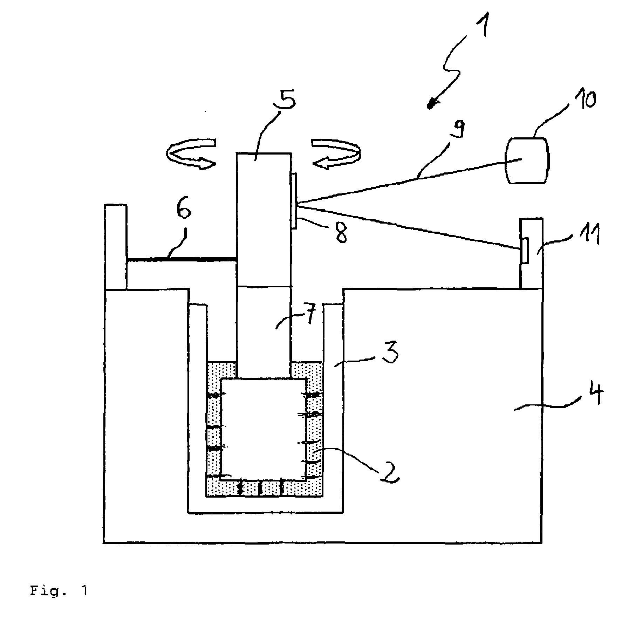

- Fig. 1: is a schematic drawing of a device for coagulation diagnosis according to a first embodiment of the invention;
- Fig. 2: is a schematic arrangement of a high-frequency plasma device usable for carrying out the method according the second embodiment of the invention;
- Fig. 3A: is a schematic drawing of fibrin and thrombocyte anchoring on a polymer surface before surface plasma treatment;
- Fig. 3B: is a schematic drawing of fibrin and thrombocyte anchoring on a polymer surface after surface plasma treatment;
- Fig. 4A to 4F: show the surface texture of different plasmatreated and untreated polymeric surfaces (A: PTFE before plasma treatment; B: PTFE after plasma treatment, C: POM before plasma treatment; D: POM after plasma treatment; E: Cyrolite® before plasma treatment; F: Cyrolite® after plasma treatment);
- Fig. 5:: is a graph showing the CL160 index of plasma treated cups and probes (right) in comparison to untreated parts (left);
- Fig. 6A and 6B: are diagrams showing a thromboelastometric measurement with plasma-treated cup and probe (A) and with untreated cup and probe (B); and
- Fig. 7: is a schematic drawing of a artery sealing according to the second embodiment of the invention.

In the following the invention is explained considering a first embodiment of the invention. According to a first embodiment of the invention there is provided a device 1 for measuring coagulating properties of a blood sample 2.

The device 1 includes a cup 3 which is basically cylindrical in shape. The cup 3 is attached to a base 4 such that it is stationary and can not move relative to the base 4.

shaft 5 is connected to the base 4 for example by a ball bearing such that it is rotatable relative to the cup. A elastic element (fine metal spring) 6 provides a restoring force for the rotational movement of the shaft relative to the cup and base. Detachably connected to the shaft is a probe 7 which has basically a cylindrical outer shape. During the measurement the probe is placed in the inside of the cup such that the blood sample 2 is between the probe and the inner sidewall of the cup.

A mirror is attached to the sidewall of the shaft to reflect a light beam 9 from a light source 10 towards a photo detector 11 so that the rotational position of the shaft 5 can be detected with high precision.

The use of a ball bearing eliminates the high susceptibility to shocks and vibration and other problems of coagulation diagnosis tools wherein the pin is suspended into the cup 3.

In operation the cup is stationary and the probe 7 is rotated back and forth by the elastic element in a range of about ±5°. When the sample in the cup 3 starts to clot, it attaches to the surfaces of the probe 7 and the cup. Thereby the blood clot forms a coupling between the cup 3 and the probe 7 or the shaft 5 which is connected to the probe 7. If a coupling forms between the probe 7 and the cup 3 by means of blood clotting, a torque acts against the rotational movement of the shaft 5 so that the shaft 5 is no longer completely rotated.

The cup 2 and the probe 7 are basically cylindrical in shape wherein the outer diameter of the probe 7 is smaller than the inner diameter of the cup 3 such that the probe 7 can be placed inside of the cup 3.

The cup 2 and probe 7 according to the invention are made of a polymer, preferably Cyrolite®. Cyrolite® has the advantage that it does not affect coagulation activation neither before nor after plasma treatment, because an affected coagulation activation as for example disclosed in US 5,334,611 1 would lead to a falsification of the measurement results.

According to the invention the method described below was used in the steps of manufacturing the cup 3 and the probe 7.

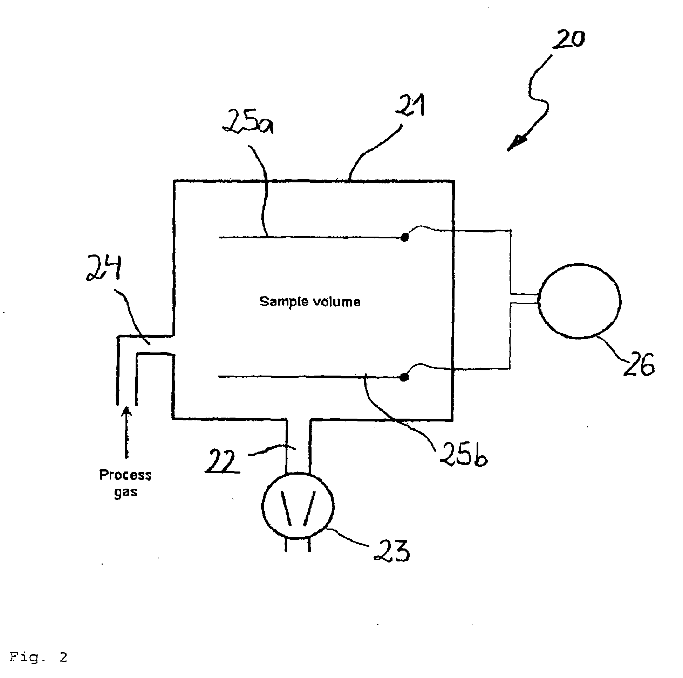

The surface of the cup 3 and the probe 7 is treated in a plasma chamber system 20 as shown in Fig. 2. The plasma chamber system comprises a recipient 21 having an outlet 22 connected to a vacuum pump 23 via a not shown valve and having an inlet 24 connected to a not shown process gas supply. In the recipient are provided two electrodes 25a and 25b which are connected to a HF generator 26. In between the two electrodes is the sample volume where the cup 3 and probe 7 are placed to be treated by the plasma, generated by the HF generator 26 in between the two electrodes 25a, 25b.

In a particular example the plasma surface treatment was carried out with the following parameters:
- Temperature T = 293 K (room temperature)
- pressure p = 1 mbar
- process time t = 60 minutes
- power density per volume of the probe P/V = 10 W/l
- frequency of the HF generator f = 40 kHZ
- process gas: oxygen

The plasma chamber system used for the low pressure plasma surface treatment is a commercial Tetra-100 system from Diener electronic, Nagold, Germany.

cup 3 and probe 7 are loaded through a not shown load lock into the above mentioned recipient 21 in the sample volume. Then the recipient 21 is evacuated to pressures below 1 mbar. After evacuation, oxygen is introduced into the recipient 21 as a process gas. The pressure in the recipient 21 is then controlled to a constant value p = 1 mbar and an electrical discharge with a frequency f = 40 kHz is ignited. The plasma treatment is continued for a time t = 60 minutes. After that the cup 3 or probe 7 is removed from the recipient 21 through the not shown load lock.

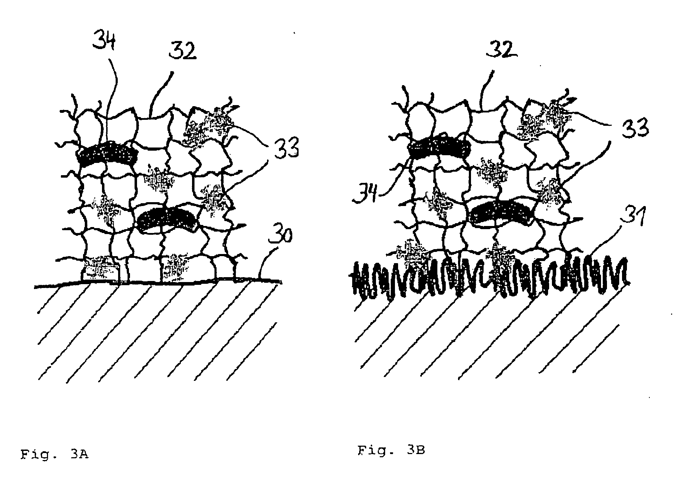

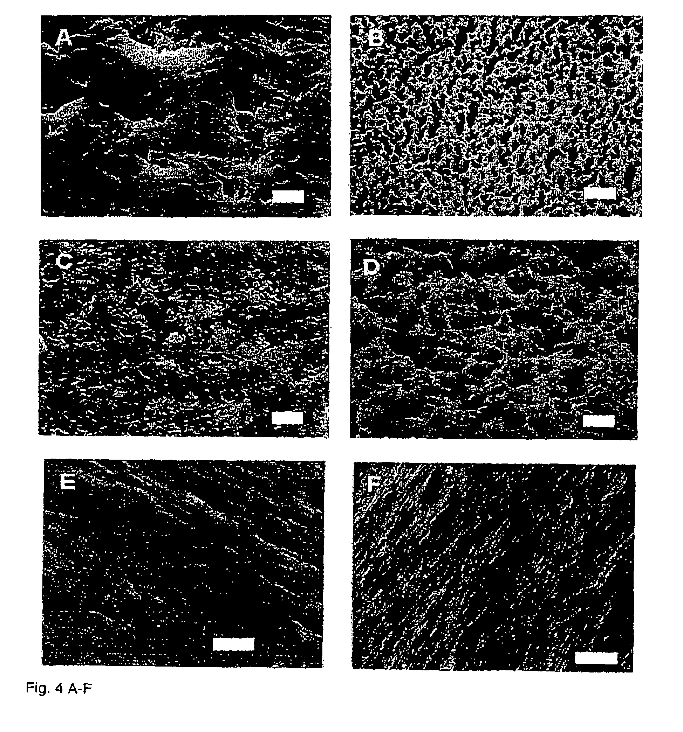

A first effect of the above described surface treatment according to the invention is that the surface texture of the cup 3 and the probe 7 is such that on a microscopic range the roughness can be characterized by an average peak-to-peak distance of 50 to 250 nm (nanoroughness) as measured between neighbored local maxima on electron microscopy images after the surface treatment (compare Fig. 4E and 4F) correspond roughly to a roughness average Ra = 10-50 nm, where Ra is defined according to the ASME standard B46.1-2002. The roughness of the surface before the described surface treatment is superposed by the before mentioned nanoroughness. An average peak to peak distance of 50 to 250 nm corresponds roughly to an roughness average Ra = 10 to 50 nm (measured according to the ASME standard B46.1-2002). Fig. 3A is a schematic drawing of the adhesion of coagulated blood on an untreated surface 30, whereas Fig. 3B is a schematic drawing of the adhesion of coagulated blood on a plasma treated surface 31. There are shown fibrin strands 32, activated thrombocytes 33, and erythrocytes 34 of coagulated blood in Figures 3A or 3B, respectively. Due to the fact that the peak to peak distance of the surface roughness is in the range of the dimension of the fibrin strands (length of the fibrin strands is about 50 nm), the surface texture obtained by the surface plasma treatment provides ideal conditions for the adhesion of fibrin strands and activated thrombocytes.

In Figures 4A to 4F the effect of the plasma treatment on different polymer materials is shown. The white bar in the Figures 4A to 4F corresponds to 3 µm. Fig. 4A shows the surface texture of PTFE before the plasma treatment and Fig. 4B the same after the plasma treatment. Fig. 4C shows the surface texture of POM before the plasma treatment and Fig. 4D the same after the plasma treatment. Fig. 4E shows the surface texture of Cyrolite® before the plasma treatment and Fig. 4F the same after the plasma treatment. It can be seen from Figures 4B, 4D and 4F that the detailed surface texture after the plasma treatment according to the invention can show rather different shapes in dependence on the used polymer material.

The detailed surface texture after plasma treatment according to the invention can differ also very much in dependence on the used process gas.

A second effect of the above described plasma treatment according to the invention is a strong cleaning effect. Contaminations of the surface are either etched away by pure mechanical interaction with the atoms or molecules in the plasma or even "burned" by chemical reactions with radicals. This surface cleaning is of essential importance with respect to quality management needs for medically used plastic parts: Due to a rather perfect surface purity after plasma treatments independent from the preceding production step(s), any further cost-intensive purification procedures can be omitted. The distribution ratio between etching and burning of contaminations mainly depends on the used process gas, but has only minor influence on the surface properties.

A third effect of the plasma surface treatment according to the invention is the generation of cover layers of incorporated gas molecules or ions. The existence of additional surface charges at the surface provides an increased number of anchoring points for activated fibrin strands or thrombocytes. However, for haemostasis analysis devices the plasma molecules must be carefully selected with respect to the used polymer material to prevent accelerated coagulation activation, since this behavior would falsify the obtained results.

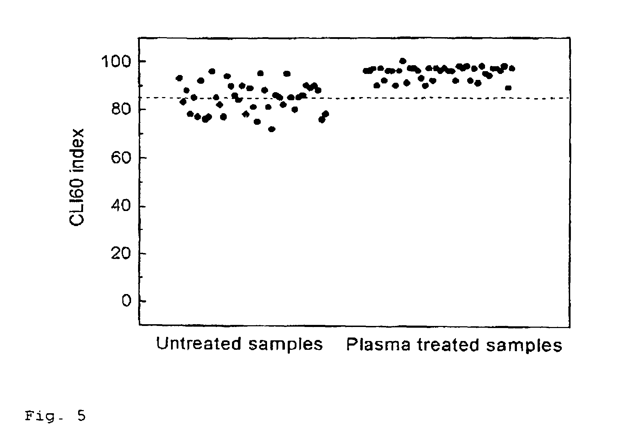

A fourth effect of the above described surface treatment according to the first embodiment of the invention is shown in Fig. 5.

Fig. 5 shows the ratio of the clot firmness measured after 60 minutes to the maximum clot firmness for normal (i.e. non-hyperfibrinolytic) donor blood samples. This ratio, also called CLI60 index, is lowered by partial ruptures of the fibrin network from the surface of the cup or the probe during measurements. Therefore, the CLI60 index is a good indicator for the adhesion strength of coagulated blood on the surface of the cup and the probe.

In total, 20 different donor samples have been measured in dublicate after the addition of thrombocytes (platelets) to a final concentration of about 800.000/µl in a device for coagulation diagnosis as described above. A first set of measurements was carried out using untreated cup and probe. A second set of measurements was carried out using treated cup and probe. The lower values of the CLI60 index for measurements using untreated cup and probe indicate partial ruptures of fibrin strands from the surface of cup and probe. The measured CLI60 index using treated cup and probe for the same blood samples are higher and therefore indicate a better adhesion of the coagulated blood to the surface of the cup and the probe.

Values below 85% (dashed line in Fig. 5) are commonly indicative for hyperfibronolitic malfunctions of the haemostasis balance. Therefore, it is important to measure values above 85% for normal blood samples without any malfunction. Only in that case a hyperfibronolitic malfunction can be determined reliably by coagulation diagnosis. The value of the CLI60 index was above 85% for all measurements using treated cup and probe, whereas for the measurements using untreated cup and probe many measurements gave values below 85%.

Since the CLI60 index (or the CLI45 index obtained after 45min) is often used for the diagnosis of hyperfibrinolysis, lower values due to insufficient surface adhesion of the fibrin network are a potential risk in haemostasia analysis. Since the blood of patients with considerably increased platelet content tends to tear off the surface due to a denser clot packing, mistakable measurements cannot be excluded. This danger could now be satisfactorily eliminated by applying plasma treatments for the disposables used in this method.

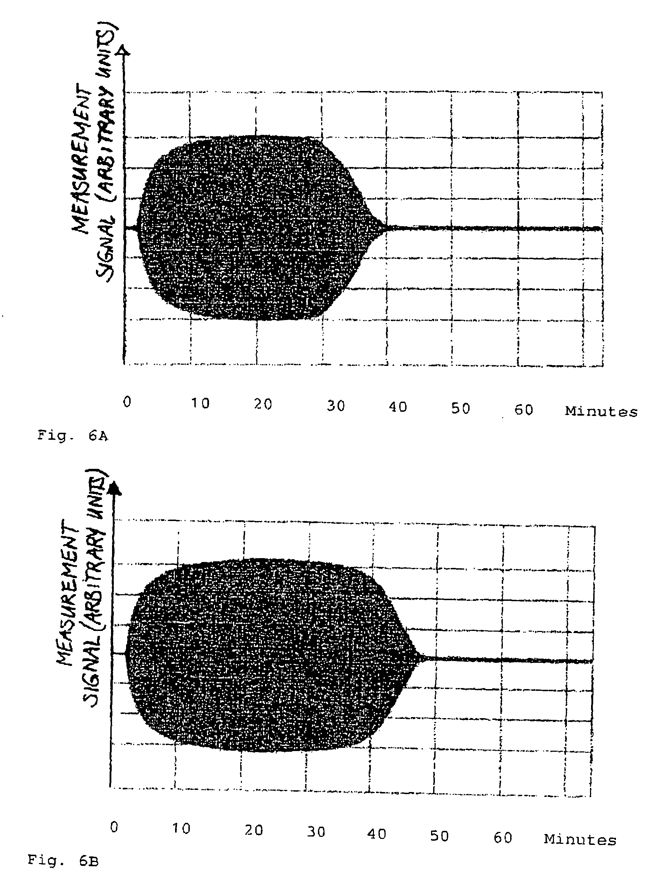

A fifth effect of the plasma treatment described above is revealed for samples in which hyperfibrinolysis is artificially induced by adding urokinase. Identical blood samples were treated with urokinase to simulate hyperfibrinolytic behavior. The result of a measurement using cup and probe with a treated surface is shown in Fig. 6A, whereas the result of a measurement using cup and probe with untreated surface is shown in Fig. 6B. In the case of using cup and probe according to the first embodiment, the pathological pattern of hyperfibrinolysis is accelerated by about 10 to 15 min compared to the measurement using cup and probe without any surface treatment and enables in this way faster medical action, e.g. in critical surgery situations.

Accordingly, the method disclosed here is highly capable to improve the coagulation analysis in a multiple manner.

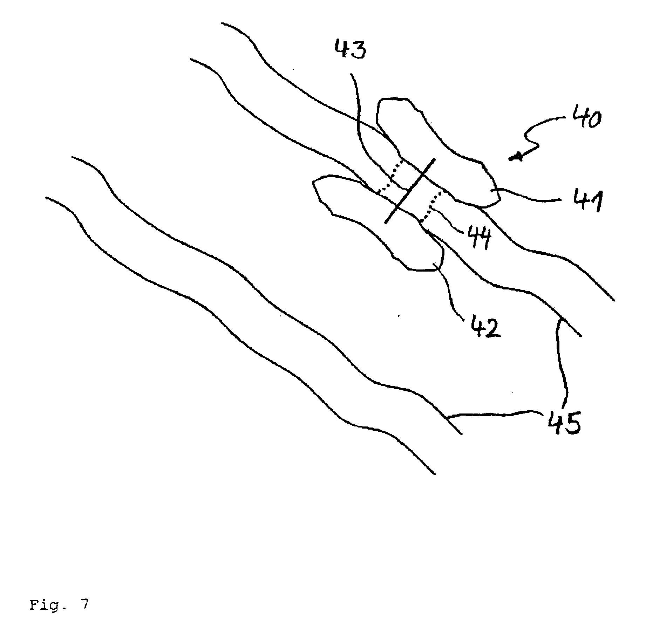

An artery sealing according to a second embodiment of the invention can be seen in Fig. 7. The artery sealing 40 comprises an outer sealant 41 and an inner sealant 42. The outer sealant 41 and the inner sealant 42 are connected by a connection piece 43 and sandwich an arteriotomy (artery puncture or injury) 44 to create haemostasis of a punctured artery 45.

The inner sealant 42 is placed within the artery 45 whereas the outer sealant 41 is placed opposite to the inner sealant 42 outside of the artery 45. The inner and outer sealant 42, 41 are preferably made of a bio-absorbable polymer (e.g. collagen, polyestheramide, polyglycolic acid, polylactic acid, polydioxanone) and treated by the above described surface plasma treatment to obtain a good sealing effect. The plasma treatment reduces considerably the probability of blood clot ruptures from the sealing which can cause embolism or apoplexy.

In the second embodiment, the same effects are obtained by the plasma treatment according to the invention as in the above described first embodiment.

Although most polymeric surfaces show in general a good ability to bind blood-inherent coagulation components like thrombocytes and fibrin, the maximum tearing force is not adequate in the case of higher blood pressures or vascular motions. In view of this, improvements of the blood anchoring ability at artificial surfaces enable the use of highly sufficient vascular sealings.

Cup and probe according to the first embodiment of the description are described to be preferably made from Cyrolite®. However, it could be also made from any other polymer which does not affect coagulation activation even after plasma treatment. Further examples for such polymers are polyethylene or acrylic plastic (e.g. polymethyl methacrylate). It was described to generate a nanoroughness with an average peak to peak distance between 50 and 250 nm. However, any nanoroughness with an average peak to peak distance below 500 nm could be used to increase the adhesion strength of coagulated blood or coagulated blood components and can be produced by the above mentioned method in dependence of the employed discharge intensity and treatment time. An average peak to peak distance below 500 nm corresponds roughly to an roughness average Ra below 100 nm (measured according to the ASME standard B46-1-2002).

The cup and probe are described to be basically cylindrical in shape and having basically a cylindrical outer shape, respectively- However, as the person skilled in the art will appreciate other shapes of the cup and/or probe are within the scope of the present invention. In particular, in certain embodiments, any shape of the cup and probe that allows (1) the probe to be placed inside of the cup and (2) the inner side wall of the cup to act as a functional surface of the device which is to be coupled to coagulated blood or to coagulated blood components is within the scope of the present invention. In particular, any cup/probe shape combination that allows blood placed between the inner sidewall of the cup and the probe to form, upon clotting, a coupling between cup and the probe as described elsewhere herein is within the scope of the present invention.

The artery sealing according to the second embodiment was described as an example for a biomaterial being treated with the plasma treatment according to the invention. However, the plasma treatment can be used for any other biomaterial for medical applications wherein the surface properties of the biomaterial must allow a sufficient adhesion of coagulated or coagulating blood. In the case of implants the blood clot serves as a scaffold for tissue regeneration.

The surface plasma treatment according to the above-described particular example of the invention was described using oxygen as a process gas. However, several other process gases could be used and further examples are: nitrogen (N₂), rare gases (e.g. Ar), air, H₂O, a CF₄/O₂-mixture or any mixtures thereof.

The surface plasma treatment according to the above described particular example of the invention was described to be carried out at low pressure (1 mbar). However, it is also possible to apply plasma treatment in any other pressure regime. Preferably the process pressure is in a range between 0.5 mbar and 5 bar.

The surface plasma treatment according to the above described particular example of the invention was described to be applied for a process time of 60 min. However, other process times are possible. The above described effects of the plasma treatment are more intense as the process time is increased. Longer process times result in higher manufacturing costs. Preferably the process time is in a range between 15 minutes and 12 hours.

Other power densities of the plasma are possible. The higher the power density of the plasma the shorter times are needed to obtain the desired effects described above. However, too high power densities will result in melting or destroying the treated parts. Preferably, the power density is in a range between 1 W/l and 100 W/l.

The surface plasma treatment according to the invention is not limited to using a HF plasma having a frequency of 40 kHz. Using a plasma with any other frequency is possible (e.g. radio frequency 13.56 MHz or microwave frequency 2.45 GHz or even a static electrical field). Preferably, the plasma frequency is in a range between 1 kHz and 10 GHz.

The surface plasma treatment according to the invention is not limited to plasma treatment carried out at 293 K. Other temperatures can be used. Higher temperatures will lead to a better outgassing of the parts to treat while lower temperatures reduce the arising gas amount. Preferably, the process temperature is in a range between 200 K and 360 K.

It is described above to change the chemical activity, textural structure and aseptic purity of a polymeric surface by plasma treatment. However, it is also possible to change the chemical activity, textural structure and aseptic purity of a polymeric surface by the treatment of the surface with an UV laser activated process gas instead of a plasma activated process gas.

It was described to modify a surface by incorporating additional charges, atoms, molecules, ions or functional groups into the surface to provide an increased number of anchoring points for activated fibrin strands or thrombocytes. However. It is also possible but not according to the invention to incorporate functional spots enhancing the adhesion of coagulated blood into the surface by chemical methods instead of by treatment by ionized atoms or molecules.

According to the above-described method of surface plasma treatment, one part is exposed to the plasma in the plasma chamber system at a time- However, it is also possible to treat more than one single part at a time, for example by employing one or several trays or a continuously rotating drum in the plasma chamber system.

## Claims

1. A method of treating the surface (30) of a device (3, 7) which is to be coupled to a clot of blood or to a clot of blood components, **characterized in that** the method comprises the step of applying a electrochemical surface treatment by exposing said surface (30) to ionized atoms or molecules such that the surface adhesion strength of coagulated blood or coagulated blood components (32, 33, 34) on said surface (31) is increased compared with the untreated surface (30).

2. The method according to claim 1 wherein said ionized atoms or molecules are generated by a high frequency electrical discharge or static electrical discharge.

3. The method according to claim 1 or 2 wherein electrical discharges with a frequency between 1 kHz and 100 GHz are applied, more preferably between 10 kHz and 10 GHz.

4. The method according to claim 1 wherein the ionized atoms and molecules are generated by an UV laser.

5. The method according to one of claims 1 to 4 wherein Ar, O₂, N₂, H₂O, air, a rare gas or any mixture thereof is used as process gas during the step of exposing said surface to ionized atoms or molecules.

6. The method according to one of claims 1 to 5 wherein said surface is exposed to ionized atoms or molecules for 15 to 500 minutes.

7. The method according to one of claims 1 to 6 wherein the step of exposing said surface to ionized atoms is carried out at a process pressure in the range of 0.1 to 5 mbar.

8. The method according to one of claims 1 to 7 wherein the step of exposing said surface to ionized atoms is carried out at room temperature.

9. The method according to one of claims 1 to 7, wherein the surface treatment is such that the surface texture of said device is modified.

10. The method according to claim 9, wherein the surface treatment is such that the modified surface texture is **characterised by** an average peak to peak distance in a range of 25 to 500 nm, preferably in a range of 50 to 250 nm.

11. The method according to one of claims 1 to 10, wherein the surface treatment is such that impurities on said surface are eliminated.

12. The method according to one of claims 1 to 11, wherein said device is a cup (3) used for analyzing coagulation properties of blood (2) or blood components or a part of such cup.

13. The method according to one of claims 1 to 12, wherein said device is a probe (7) used for analyzing coagulation properties of blood or blood components or a part of such probe.

14. The method according to one of claims 1 to 13, wherein said device is made of a biomaterial and used for implantation into the body of a human or animal or a biomaterial used for vascular surgery or other vascular treatment.

15. The method according to one of claims 1 to 14, wherein the device is made of polymer which does not affect the coagulation activity of blood or blood components, preferably a polyethylene or acrylic plastic, preferably polymethyl methacrylate.

16. A device which is to be coupled to coagulated blood or to coagulated blood components, **characterized in that** the surface properties of at least a part of the surface of the device (3, 7) are modified by exposure to ionized atoms or molecules according to claim 1 to enhance the adhesion strength of coagulated blood or coagulated blood components to the surface (31) in comparison to the untreated surface (30).

17. The device according to claim 16, wherein the average peak to peak distance of the surface roughness generated by the exposure to ionized atoms or molecules is between 25 and 500 nm, more preferably between 50 and 250 nm.

18. The device according to claim 16 or 17 wherein the surface average roughness Ra is below 100 nm, more preferably below 80 nm.

19. The device according to one of claims 16 to 18 wherein the device is a cup or a probe used for hemostasis analysis or a part of such cup or such probe.

20. The device according to one of claims 16 to 18 wherein the device is a biomaterial used for implantation into the body of a human or an animal or used in vascular surgery or other vascular treatment

21. The device according to one of claims 16 to 20 wherein the device is made of a polymer, preferably a polyethylene or acrylic plastic, preferably polymethyl methacrylate.

22. The device according to claim 21, wherein the polymer does not affect the coagulation activity of blood or blood components.

23. The device for hemostasis analysis including a cup (3) and/or a probe (7) and/or a part of a cup and/or a part of a probe according to claim 19.

24. The device according to one of claims 16 to 23 which is to be coupled to coagulated blood or to coagulated blood components, **characterized in that** the surface roughness average Ra is between 5 to 100 nm, more preferably between 5 to 80 nm.

## Patentansprüche

1. Verfahren zur Behandlung der Oberfläche (30) einer Vorrichtung (3, 7), die an ein Blutgerinnsel oder an ein Gerinnsel aus Blutbestandteilen gekoppelt werden soll, **dadurch gekennzeichnet, dass** das Verfahren den Schritt der Verabreichung einer elektrochemischen Oberflächenbehandlung umfasst, bei dem die Oberfläche (30) ionisierten Atomen oder Molekülen ausgesetzt wird, so dass die Haftfestigkeit der Oberfläche von geronnenem Blut oder geronnenen Blutbestandteilen (32, 33, 34) auf der Oberfläche (31) im Vergleich zu der unbehandelten Oberfläche (30) erhöht ist.

2. Verfahren nach Anspruch 1, worin die ionisierten Atome oder Moleküle durch eine elektrische Hochfrequenz-Entladung oder eine statische elektrische Entladung erzeugt werden.

3. Verfahren nach Anspruch 1 oder 2, worin elektrische Entladungen mit einer Frequenz zwischen 1 kHz und 100 GHz angelegt werden, insbesondere zwischen 10 kHz und 10 GHz.

4. Verfahren nach Anspruch 1, worin die ionisierten Atome und Moleküle von einem UV-Laser erzeugt werden.

5. Verfahren nach einem der Ansprüche 1 bis 4, worin Ar, O₂, N₂, H₂O, Luft, ein Edelgas oder eine beliebige Mischung davon als Verfahrensgas bei dem Schritt verwendet wird, bei dem die Oberfläche ionisierten Atomen oder Molekülen ausgesetzt wird.

6. Verfahren nach einem der Ansprüche 1 bis 5, worin die Oberfläche 15 bis 500 Minuten ionisierten Atomen oder Molekülen ausgesetzt wird.

7. Verfahren nach einem der Ansprüche 1 bis 6, worin der Schritt, bei dem die Oberfläche ionisierten Atomen ausgesetzt wird, bei einem Verfahrensdruck im Bereich von 0,1 bis 5 mbar ausgeführt wird.

8. Verfahren nach einem der Ansprüche 1 bis 7, worin der Schritt, bei dem die Oberfläche ionisierten Atomen ausgesetzt wird, bei Raumtemperatur ausgeführt wird.

9. Verfahren nach einem der Ansprüche 1 bis 7, worin Oberflächenbehandlung so beschaffen ist, dass die Oberflächenstruktur der Vorrichtung modifiziert wird.

10. Verfahren nach Anspruch 9, worin die Oberflächenbehandlung so beschaffen ist, dass die modifizierte Oberflächenstruktur durch einen durchschnittlichen Spitzen-Spitzen-Abstand im Bereich von 25 bis 500 nm, vorzugsweise im Bereich von 50 bis 250 nm **gekennzeichnet** ist.

11. Verfahren nach einem der Ansprüche 1 bis 10, worin die Oberflächenbehandlung so beschaffen ist, dass Verunreinigungen auf der Oberfläche beseitigt werden.

12. Verfahren nach einem der Ansprüche 1 bis 11, worin die Vorrichtung ein Becher (3) zur Untersuchung der Gerinnungseigenschaften von Blut (2) oder von Blutbestandteilen oder ein Teil eines solchen Bechers ist.

13. Verfahren nach einem der Ansprüche 1 bis 12, worin die Vorrichtung eine Sonde (7) zur Untersuchung der Gerinnungseigenschaften von Blut oder von Blutbestandteilen oder ein Teil einer solchen Sonde ist.

14. Verfahren nach einem der Ansprüche 1 bis 13, worin die Vorrichtung aus einem biologischen Material besteht und zur Implantation im Körper eines Menschen oder eines Tieres verwendet wird oder aus einem biologischen Material besteht, das für die Gefäßchirurgie oder eine andere Gefäßbehandlung verwendet wird.

15. Verfahren nach einem der Ansprüche 1 bis 14, worin die Vorrichtung aus einem Polymer besteht, das die Gerinnungsaktivität von Blut oder Blutbestandteilen nicht beeinflusst, vorzugsweise aus Polyethylen oder einem Acrylkunststoff, vorzugsweise aus Polymethylmethacrylat.

16. Vorrichtung, die an geronnenes Blut oder an geronnene Blutbestandteile gekoppelt werden soll, **dadurch gekennzeichnet, dass** die Oberflächeneigenschaften zumindest eines Teils der Oberfläche der Vorrichtung (3, 7) durch Exposition gegenüber ionisierten Atomen oder Molekülen nach Anspruch 1 modifiziert wird, um die Haftfestigkeit von geronnenem Blut oder von geronnenen Blutbestandteilen an der Oberfläche (31) im Vergleich zur unbehandelten Oberfläche (30) zu erhöhen.

17. Vorrichtung nach Anspruch 16, worin der durchschnittliche Spitzen-Spitzen-Abstand der Oberflächenrauhigkeit, der durch die Exposition gegenüber den ionisierten Atomen oder Molekülen erzeugt wird, zwischen 25 und 500 nm, insbesondere zwischen 50 und 250 nm beträgt.

18. Vorrichtung nach Anspruch 16 oder 17, worin die durchschnittliche Oberflächenrauhigkeit Ra unter 100 nm, insbesondere unter 80 nm liegt.

19. Vorrichtung nach einem der Ansprüche 16 bis 18, worin die Vorrichtung ein Becher oder eine Sonde zur Hämostase-Untersuchung oder ein Teil eines solchen Bechers oder einer solchen Sonde ist.

20. Vorrichtung nach einem der Ansprüche 16 bis 18, worin die Vorrichtung ein biologisches Material zur Implantation in den Körper eines Menschen oder eines Tieres oder für die Gefäßchirurgie oder eine andere Gefäßbehandlung ist.

21. Vorrichtung nach einem der Ansprüche 16 bis 20, worin die Vorrichtung aus einem Polymer, vorzugsweise aus Polyethylen oder einem Acryl-Kunststoff, vorzugsweise aus Polymethylmethacrylat besteht.

22. Vorrichtung nach Anspruch 21, worin das Polymer die Gerinnungsaktivität von Blut oder Blutbestandteilen nicht beeinflusst.

23. Vorrichtung zur Hämostase-Untersuchung mit einem Becher (3) und/oder einer Sonde (7) und/oder einem Teil eines Bechers und/oder einem Teil einer Sonde nach Anspruch 19.

24. Vorrichtung nach einem der Ansprüche 16 bis 23, die an geronnenes Blut oder an geronnene Blutbestandteile gekoppelt werden soll, **dadurch gekennzeichnet, dass** die durchschnittliche Oberflächerauhigkeit Ra zwischen 5 und 100 nm, insbesondere zwischen 5 und 80 nm liegt.

## Revendications

1. Procédé de traitement de la surface (30) d'un dispositif (3, 7) qui est couplé à un caillot de sang ou à un caillot de composants sanguins, **caractérisé en ce que** le procédé comprend l'étape d'application d'un traitement de surface électrochimique en exposant ladite surface (30) à des atomes ou molécules ionisés de sorte que la force d'adhérence à la surface de sang coagulé ou de composants sanguins coagulés (32, 33, 34) sur ladite surface (31) est augmentée comparativement à la surface non traitée (30).

2. Procédé selon la revendication 1, dans lequel lesdits atomes ou molécules ionisés sont générés par une décharge électrique à haute fréquence ou une décharge d'électricité statique.

3. Procédé selon la revendication 1 ou la revendication 2, dans lequel des décharges électriques avec une fréquence située en 1 kHz et 100 GHz sont appliquées, plus préférablement située entre 10 kHz et 10 GHz.

4. Procédé selon la revendication 1, dans lequel lesdits atomes ou molécules ionisés sont générés par un laser UV.

5. Procédé selon l'une quelconque des revendications 1 à 4, dans lequel de l'Ar, de l'O₂, du N₂, de l'H₂O, de l'air, un gaz rare ou tout mélange de ceux-ci est utilisé comme gaz de traitement pendant l'étape d'exposition de ladite surface à des atomes ou molécules ionisés.

6. Procédé selon l'une quelconque des revendications 1 à 5, dans lequel ladite surface est exposée à des atomes ou molécules ionisés pendant 15 à 500 minutes.

7. Procédé selon l'une quelconque des revendications 1 à 6, dans lequel l'étape d'exposition de ladite surface à des atomes ou molécules ionisés est réalisée à une pression de traitement située dans la plage de 0,1 à 5 mbar.

8. Procédé selon l'une quelconque des revendications 1 à 7, dans lequel l'étape d'exposition de ladite surface à des atomes ionisés est réalisée à la température ambiante.

9. Procédé selon l'une quelconque des revendications 1 à 7, dans lequel le traitement de la surface est tel que la texture de la surface dudit dispositif est modifiée.

10. Procédé selon la revendication 9, dans lequel le traitement de la surface est tel que la texture de la surface modifiée est **caractérisée par** une distance moyenne pic à pic située dans une plage de 25 à 500 nm, de préférence dans une plage de 50 à 250 nm.

11. Procédé selon l'une quelconque des revendications 1 à 10, dans lequel le traitement de la surface est tel que les impuretés sur ladite surface sont éliminées.

12. Procédé selon l'une quelconque des revendications 1 à 11, dans lequel ledit dispositif est une coupe (3) utilisée pour l'analyse des propriétés de coagulation du sang (2) ou de composants sanguins ou une partie d'une telle coupe.

13. Procédé selon l'une quelconque des revendications 1 à 12, dans lequel ledit dispositif est une sonde (7) utilisée pour l'analyse des propriétés de coagulation du sang ou de composants sanguins ou une partie d'une telle sonde.

14. Procédé selon l'une quelconque des revendications 1 à 13, dans lequel ledit dispositif est constitué d'un biomatériau et utilisé pour une implantation dans le corps d'un être humain ou d'un animal ou un biomatériau utilisé pour une chirurgie vasculaire ou autre traitement vasculaire.

15. Procédé selon l'une quelconque des revendications 1 à 14, dans lequel ledit dispositif est constitué d'un polymère qui ne perturbe pas l'activité de coagulation du sang ou de composants sanguins, de préférence un plastique polyéthylène ou acrylique, de préférence du méthacrylate de polyméthyle.

16. Dispositif qui doit être couplé à du sang coagulé ou à des composants sanguins coagulés, **caractérisé en ce que** les propriétés de surface d'au moins une partie de la surface du dispositif (3, 7) sont modifiées par exposition à des atomes ou molécules ionisés selon la revendication 1 pour améliorer la force d'adhérence de sang coagulé ou de composants sanguins coagulés à la surface (31) comparativement à la surface non traitée (30).

17. Dispositif selon la revendication 16, dans lequel la distance moyenne pic à pic de la rugosité de la surface générée par l'exposition à des atomes ou molécules ionisés se situe entre 25 et 500 nm, plus préférablement entre 50 et 250 nm.

18. Dispositif selon la revendication 16 ou la revendication 17, dans lequel la rugosité moyenne de la surface Ra est inférieure à 100 nm, de préférence inférieure à 80 nm.

19. Dispositif selon l'une quelconque des revendications 16 à 18, dans lequel le dispositif est une coupe ou une sonde utilisée pour une analyse de l'hémostase ou une partie d'une telle coupe ou d'une telle sonde.

20. Dispositif selon l'une quelconque des revendications 16 à 18, dans lequel le dispositif est un biomatériau utilisé pour une implantation dans le corps d'un être humain ou d'un animal ou utilisé pour une chirurgie vasculaire ou autre traitement vasculaire.

21. Dispositif selon l'une quelconque des revendications 16 à 20, dans lequel le dispositif est constitué d'un polymère, de préférence un plastique polyéthylène ou acrylique, de préférence du méthacrylate de polyméthyle.

22. Dispositif selon la revendication 21, dans lequel le polymère ne perturbe pas l'activité de coagulation du sang ou de composants sanguins.

23. Dispositif pour une analyse de l'hémostase, qui comprend une coupe (3) et/ou une sonde (7) et/ou une partie d'une coupe et/ou une partie d'une sonde selon la revendication 19.

24. Dispositif selon l'une quelconque des revendications 16 à 23, ledit dispositif étant couplé à du sang coagulé ou à des composants sanguins coagulés, **caractérisé en ce que** la rugosité moyenne de la surface Ra se situe entre 5 et 100 nm, plus préférablement entre 5 et 80 nm.
